(19) Europäisches Patentamt
European Patent Office
Office européen des brevets

(11) Veröffentlichungsnummer: **0 290 904**
**A1**

# (12) EUROPÄISCHE PATENTANMELDUNG

(21) Anmeldenummer: **88107011.4**

(51) Int. Cl.⁴: **C07D 231/16 , A01N 43/56**

(22) Anmeldetag: **02.05.88**

(30) Priorität: **12.05.87 DE 3715703**

(43) Veröffentlichungstag der Anmeldung:
**17.11.88 Patentblatt 88/46**

(84) Benannte Vertragsstaaten:
**BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Heine, Hans-Georg, Dr.**
**Am Heckerhof 14**
**D-4150 Krefeld(DE)**
Erfinder: **Ooms, Pieter, Dr.**
**Doerperhofstrasse 16**
**D-4150 Krefeld(DE)**
Erfinder: **Bielefeldt, Dietmar, Dr.**
**Beuthener Strasse 13**
**D-40390 Ratingen(DE)**
Erfinder: **Becker, Benedikt, Dr.**
**Metzkausener Strasse 14**
**D-4020 Mettmann(DE)**
Erfinder: **Behrenz, Wolfgang, Dr.**
**Untergruendemich 14**
**D-5063 Overath(DE)**

(54) **1-Aryl-3-halogenalkyl-4-halogen-pyrazole.**

(57) Es werden neue 1-Aryl-3-halogenalkyl-4-halogen-pyrazole der Formel (I)

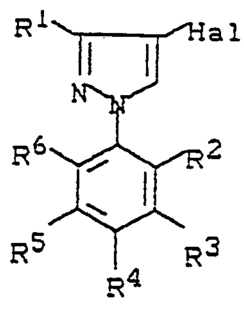

(I)

bereitgestellt,
in welcher
$R^1$ für Halogenalkyl steht,
Hal für Halogen steht und
$R^2$, $R^3$, $R^4$, $R^5$ und $R^6$ jeweils unabhängig voneinander für Wasserstoff, Halogen, Cyan, Nitro, Alkyl, Alkylthio, Alkylsulfinyl, Alkylsulfonyl, Alkoxycarbonyl oder für den Rest $-(X)_n-R^7$ stehen,
wobei
X für Sauerstoff, Schwefel, Sulfinyl oder Sulfonyl steht,
n für die Zahl 0 oder 1 steht und

EP 0 290 904 A1

$R^7$ für Halogenalkyl steht,
wobei, falls $R^2$ bis $R^6$ gleichzeitig für Wasserstoff stehen, $R^1$ nicht für Trifluormethyl und Hal nicht für Fluor stehen darf.

Die erfindungsgemäßen Verbindungen besitzen eine stark ausgeprägte insektizide Wirksamkeit.

## 1-Aryl-3-halogenalkyl-4-halogen-pyrazole

Die vorliegende Erfindung betrifft neue 1-Aryl-3-halogenalkyl-4-halogen-pyrazole, Verfahren zu ihrer Herstellung und ihre Verwendung als Insektizide.

Es ist bereits bekannt geworden, daß verschiedene 1-Arylpyrazol-Derivate pestizide Eigenschaften besitzen. So sind beispielsweise bestimmte Säureamide des 4-Cyan-5-amino-1-(2,4,6-trichlorphenyl)-pyrazols herbizid wirksam (DE-OS 3 226 513). Herbizide Wirkung besitzen ebenfalls 1-Arylpyrazol-Derivate, die durch CN in 4-Stellung und Halogen in 5-Stellung substituiert sind (DE-OS 3 520 329). Aus einer Arbeit über Reaktionen polyhalogenierter Allene ist 1-Phenyl-3-trifluoromethyl-4-fluor-pyrazol bekannt (J. Chem. Soc. 1982, 2207). Über die insektizide Wirkung dieser Verbindungen ist jedoch nichts bekannt geworden.

Es wurden neue 1-Aryl-3-halogenalkyl-4-halogen-pyrazole der Formel (1)

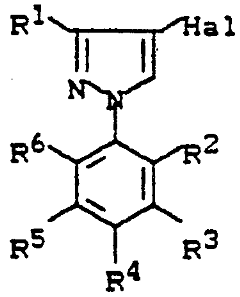

(I)

gefunden,
in welcher
$R^1$ für Halogenalkyl steht,
Hal für Halogen steht und
$R^2$, $R^3$, $R^4$, $R^5$ und $R^6$ jeweils unabhängig voneinander für Wasserstoff, Halogen, Cyano, Nitro, Alkyl, Alkylthio, Alkylsulfinyl, Alkylsulfonyl, Alkoxycarbonyl oder für den Rest $-(X)_n-R^7$ stehen,
wobei
X für Sauerstoff, Schwefel, Sulfinyl oder Sulfonyl steht,
n für die Zahl 0 oder 1 steht und
$R^7$ für Halogenalkyl steht,
wobei jedoch für den Fall, daß $R^2$ bis $R^6$ gleichzeitig für Wasserstoff stehen, $R^1$ nicht für Trifluormethyl und Hal nicht für Fluor stehen darf.

Weiterhin wurde gefunden, daß man die neuen 1-Aryl-3-halogenalkyl-4-halogen-pyrazole der Formel (I) erhält, wenn man

a) halogensubstituierte Acylenolether der Formel (II)

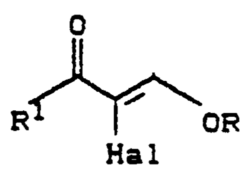

(II)

in welcher
$R^1$ für Halogenalkyl steht,
Hal für Halogen steht und
R für Alkyl steht,
mit Arylhydrazinen der Formel (III)

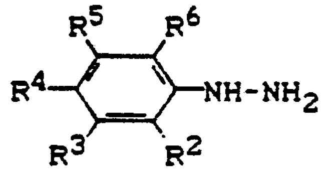

(III)

in welcher

$R^2$ bis $R^6$ die obengenannte Bedeutung haben, gegebenenfalls in einem Verdünnungsmittel umsetzt, oder wenn man

b) N-Arylhydrazid-halogenide der Formel (IV)

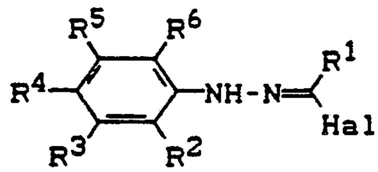

(IV)

in welcher

$R^1$ bis $R^6$ und Hal die obengenannte Bedeutung haben, mit Alkenen der Formel (V)

Hal-CH = CH-Hal (V)

in welcher

Hal die obengenannte Bedeutung besitzt, gegebenenfalls in einem Verdünnungsmittel und gegebenenfalls in Gegenwart eines säurebindenden Mittels umsetzt, oder wenn man

c) 1-Aryl-3-halogenalkyl-pyrazole der Formel (VI)

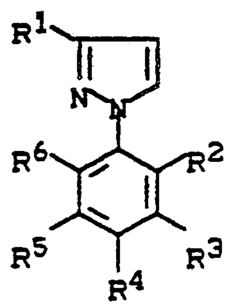

(VI)

in welcher

$R^1$ bis $R^6$ die obengenannte Bedeutung haben,

mit einem Halogenierungsmittel, wie Fluor, Chlor oder Brom, gegebenenfalls in einem Verdünungsmittel umsetzt, oder wenn man

d) N-Arylsydnone der Formel (VII)

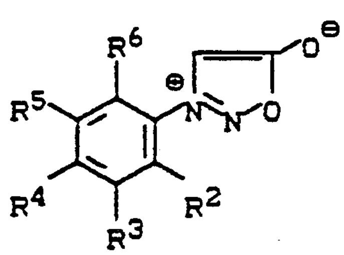

(VII)

in welcher

$R^2$ bis $R^6$ die obengenannte Bedeutung haben, mit Alkinen der Formel (VIII)

$R^1$-C≡C-Hal (VIII)

in welcher

$R^1$ und Hal die genannte Bedeutung haben,

gegebenenfalls in einem Verdünnungsmittel umsetzt.

Schließlich wurde gefunden, daß die neuen 1-Aryl-3-halogenalkyl-4-halogen-pyrazole eine stark ausgeprägte insektizide Wirkung besitzen.

Überraschenderweise zeigen die erfindungsgemäßen 1-Aryl-3-halogenalkyl-4-halogen-pyrazole der allgemeinen Formel (I) eine erheblich bessere insektizide Wirkung als die aus dem Stand der Technik bekannten Pyrazol-Derivate, wie beispielsweise das 4-Cyano-5-propionylamino-1-(2,4,6-trichlorophenyl)-pyrazol, welches aus der DE-OS 3 226 513 bekannt ist und welches eine chemisch naheliegende Verbindung ist.

Die erfindungsgemäßen 1-Aryl-3-halogenalkyl-4-halogen-pyrazole sind durch die Formel (I) allgemein definiert. Bevorzugt sind Verbindungen der Formel (I), bei welchen

$R^1$ für geradkettiges oder verzweigtes Halogenalkyl mit 1 bis 4 Kohlenstoffatomen und 1 bis 9 gleichen oder verschiedenen Halogenatomen steht,

Hal für Fluor, Chlor, Brom oder Iod steht und

$R^2$, $R^3$, $R^4$, $R^5$ und $R^6$ jeweils unabhängig voneinander für Wasserstoff, Halogen, Cyan, Nitro, Alkyl, Alkylthio, Halogenalkyl, Halogenalkoxy, Halogenalkylthio, Alkylsulfinyl, Alkylsulfonyl, Halogenalkylsulfinyl, Halogenalkylsulfonyl oder Alkoxycarbonyl mit jeweils 1 bis 4 Kohlenstoffatomen in den einzelnen Alkylteilen und gegebenfalls 1 bis 9 gleichen oder verschiedenen Halogenatomen stehen, wobei, falls $R^2$ bis $R^6$ gleichzeitig für Wasserstoff stehen, $R^1$ nicht für Trifluormethyl und Hal nicht für Fluor stehen darf.

Besonders bevorzugt sind Verbindungen der Formel (I), in der

$R^1$ für Trifluormethyl, Dichlorfluormethyl, Difluorchlormethyl, Difluormethyl, Trifluorethyl, Trifluorchlorethyl, Tetrafluorethyl, Pentafluorethyl, Heptafluorpropyl oder Nonafluorbutyl steht,

Hal für Fluor, Chlor oder Brom steht und

$R^2$, $R^3$, $R^4$, $R^5$ und $R^6$ jeweils unabhängig voneinander für Wasserstoff, Fluor, Chlor, Brom, Cyan, Nitro, Methyl, Ethyl, n-oder i-Propyl, n-, i-, s-oder tert.-Butyl, Methylthio, Methylsulfinyl, Methylsulfonyl, Methoxycarbonyl, Ethoxycarbonyl, Trifluormethyl, Dichlorfluormethyl, Difluorchlormethyl, Difluormethyl, Pentafluorethyl, Trifluorchlorethyl, Trifluorethyl, Trifluordichlorethyl, Difluorchlorethyl oder für den Rest -X-$R^7$ steht, wobei

X für Sauerstoff, Schwefel, Sulfinyl oder Sulfonyl steht und

$R^7$ für Trifluormethyl, Dichlorfluromethyl, Difluorchlormethyl, Difluormethyl, Pentafluorethyl, Tetrafluorethyl, Trifluorchlorethyl, Trifluorethyl, Difluorchlorethyl, Trichlormethyl oder Trifluordichlorethyl steht,

wobei, falls $R^2$ bis $R^6$ gleichzeitig für Wasserstoff stehen, $R^1$ nicht für Trifluormethyl und Hal nicht für Fluor stehen darf.

Außer den in den Herstellungsbeispielen genannten Verbindungen seien im einzelnen folgende 1-Aryl-3-halogenalkyl-4-halogen-pyrazole der Formel (I) aufgeführt:

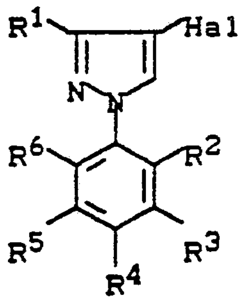

(I)

| $R^1$ | Hal | $R^2$ | $R^3$ | $R^4$ | $R^5$ | $R^6$ |
|---|---|---|---|---|---|---|
| $CF_3$ | Cl | Cl | H | $CF_3$ | H | F |
| $CF_3$ | Cl | F | F | $CF_3$ | F | F |
| $CF_3$ | Br | Cl | H | $SO_2CF_3$ | H | Cl |
| $CF_3$ | Br | Cl | H | $CF_3$ | H | F |
| $CF_3$ | F | Cl | H | Cl | H | Cl |
| $CF_3$ | F | F | F | $CF_3$ | F | F |
| $CF_3$ | Cl | Cl | H | $OCF_3$ | H | Cl |
| $CF_3$ | Cl | Cl | H | $CF_3$ | H | H |

| $R^1$ | Hal | $R^2$ | $R^3$ | $R^4$ | $R^5$ | $R^6$ |
|---|---|---|---|---|---|---|
| $CF_3$ | Br | Cl | Cl | $CF_3$ | H | Cl |
| $CF_3$ | F | Cl | Cl | $CF_3$ | H | Cl |
| $C_2F_5$ | Cl | Cl | H | $CF_3$ | H | Cl |
| $C_2F_5$ | Br | Cl | H | $CF_3$ | H | Cl |
| $C_2F_5$ | Cl | F | F | $CF_3$ | F | F |
| $CF_3$ | F | Cl | H | $CF_3$ | H | F |
| $C_2F_5$ | F | Cl | H | $CF_3$ | H | F |
| $C_2F_5$ | F | Cl | H | $CF_3$ | H | Cl |
| $C_2F_5$ | Cl | Cl | H | $SO_2CF_3$ | H | Cl |
| $C_3F_7$ | Cl | Cl | H | $CF_3$ | H | Cl |
| $C_3F_7$ | F | Cl | H | $CF_3$ | H | Cl |
| $C_3F_7$ | Br | Cl | H | $CF_3$ | H | Cl |
| $C_3F_7$ | Cl | Cl | H | $SO_2CF_3$ | H | Cl |
| $C_3F_7$ | F | Cl | H | Cl | H | Cl |
| $C_4F_9$ | Cl | Cl | H | $CF_3$ | H | Cl |
| $CCl_2F$ | Cl | Cl | H | $CF_3$ | H | Cl |
| $CF_2Cl$ | Cl | Cl | H | $CF_3$ | H | Cl |
| $C_3F_7$ | F | Cl | H | $CF_3$ | H | F |
| $CHF_2$ | Cl | Cl | H | $CF_3$ | H | Cl |
| $CHF_2$ | Cl | Cl | H | $SO_2CF_3$ | H | Cl |

Verwendet man beispielsweise 3-Brom-4-ethoxy-1,1,1-trifluor-but-3-en-2 on und 2,6-Dichlor-4-trifluormethylphenylhydrazin als Ausgangsstoffe, so kann der Reaktionsverlauf des erfindungsgemäßen Verfahrens (a) durch das folgende Schema wiedergegeben werden:

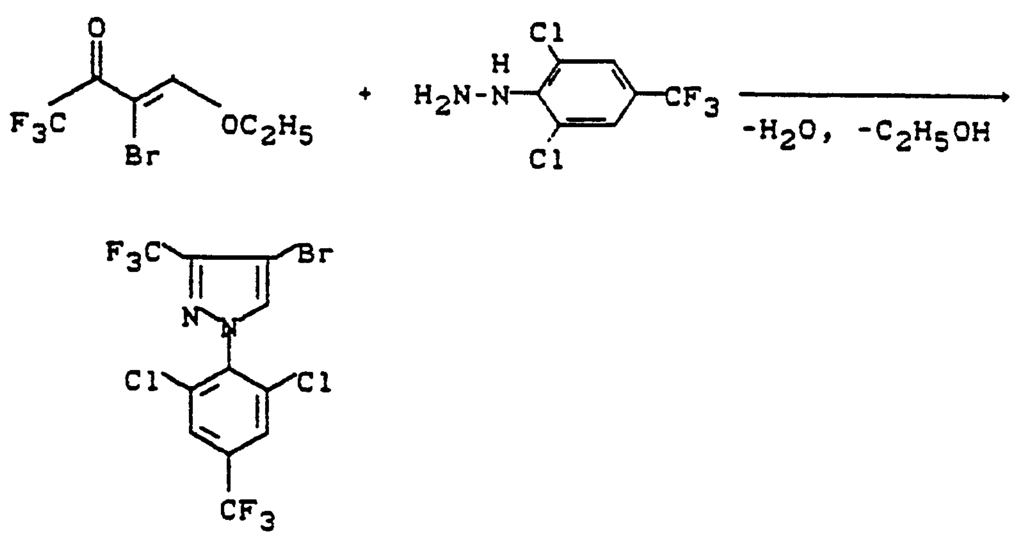

Verwendet man beispielsweise N-(2-Chlor-6-fluor-4-trifluormethylphenyl)-trifluoracethydrazid-bromid und 1,2-Dichlorethylen als Ausgangsstoffe, so kann der Reaktionsverlauf des erfindungsgemäßen Verfahrens (b) durch das folgende Formelschema wiedergegeben werden:

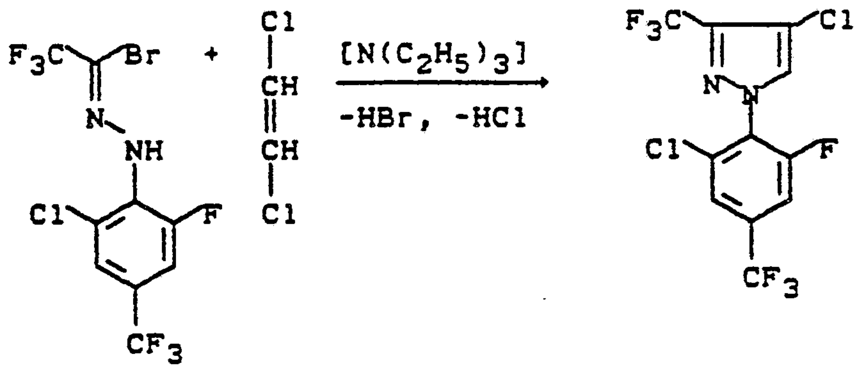


Verwendet man beispielsweise 1-(2,6-Dichlor-4-trifluormethoxyphenyl)-3-trifluormethylpyrazol und Fluor als Ausgangsstoffe, so läßt sich der Reaktionsverlauf des erfindungsgemäßen Verfahrens (c) durch das folgende Formelschema wiedergegeben:

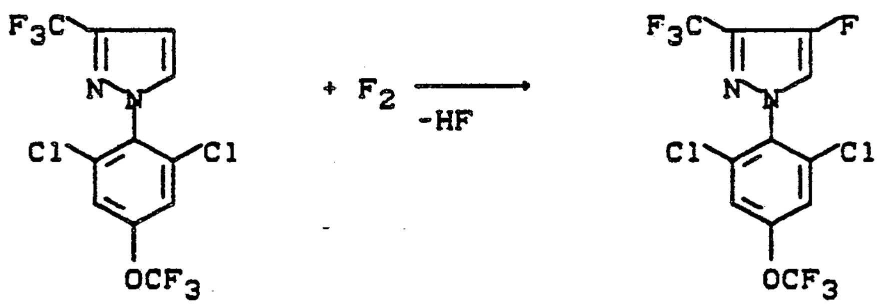


Verwendet man beispielsweise N-(2,6-Dichlor-4-trifluormethylphenyl)-sydnon und Tetrafluorpropin als Ausgangsstoffe, so läßt sich der Reaktionsverlauf des erfindungsgemäßen Verfahrens (d) durch das folgende Formelschema wiedergegeben:

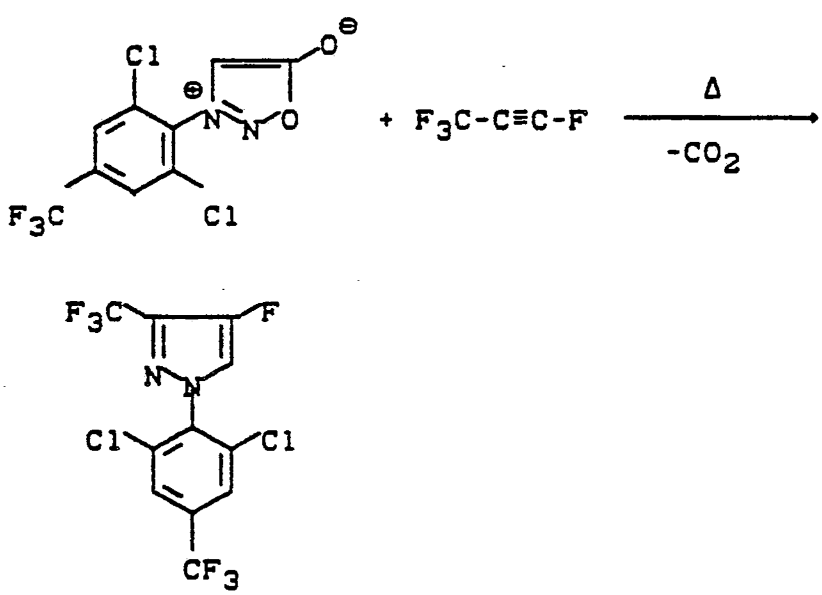


Die zur Durchführung des erfindungsgemäßen Verfahrens (a) als Ausgangsprodukte benötigten halogensubstituierten Acylenolether der Formel (II)

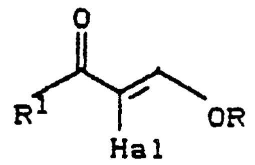

(II)

in welcher
$R^1$, Hal und R die obengenannte Bedeutung haben, sind neu und Gegenstand einer Parallelanmeldung. Man erhält sie, wenn man beispielsweise Acylenolether der Formel (IX)

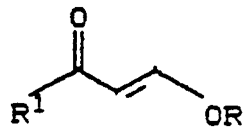

(IX)

in welcher
$R^1$ und R die obengenannte Bedeutung haben, mit einem Halogenierungsmittel bei Temperaturen von -70 °C bis +80 °C, gegebenenfalls in Gegenwart eines Verdünnungsmittels umsetzt, das resultierende Halogenierungsprodukt der Formel (X)

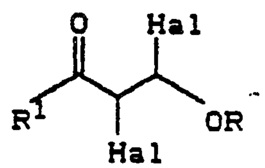

(X)

gegebenenfalls isoliert und bei einer Temperatur von -20 °C bis +100 °C dehydrohalogeniert.

Die als Ausgangsprodukte benötigten Arylhydrazine der Formel (III) sind bekannt (z.B. EP 154 115:187 285; 34 945).

Die zur Durchführung des erfindungsgemäßes Verfahrens (b) als Ausgangsstoffe benötigten N-Arylhydrazid-halogenide sind durch die Formel (IV) allgemein definiert. In dieser Formel stehen $R^1$ bis $R^6$ und Hal vorzugsweise für diejenigen Substituenten, die bereits im Zusammenhang mit der Beschreibung der erfindungsgemäßen Verbindungen der Formel (I) als bevorzugt für diese Reste genannt sind.

Die N-Arylhydrazid-halogenide der Formel (IV) sind teilweise bekannt (Chem. Lett. 1982 543; J. Heterocycl. Chem. 22, 565 (1985); EP 1 019). Man erhält sie beispielsweise, wenn man Arylhydrazine der Formel (III) mit Halogenalkylaldehyden der Formel (XI) oder deren Halbacetalen oder Hydraten (XII)

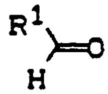

(XI)

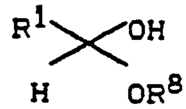

(XII)

in welcher
$R^1$ die oben angegebene Bedeutung hat und
$R^8$ für Wasserstoff oder Alkyl steht zu den Halogenalkylaldehyde-N-arylhydrazonen der Formel (XIII)

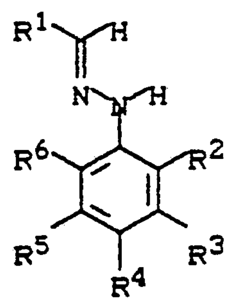

(XIII)

in welcher
$R^1$ bis $R^6$ die angegebene Bedeutung haben,
umsetzt und diese mit Halogenierungsmitteln, wie beispielsweise Bromsuccinimid, Chlorsuccinimid oder Brom, gegebenenfalls in Gegenwart eines Verdünnungsmittels, wie beispielsweise Dimethylformamid oder Essigsäure, bei Temperaturen zwischen -30 °C und 100 °C umsetzt.

Die Halogenalkylaldehyde der Formel (XI) sowie ihre Hydrate oder Acetale der Formel (XII) sind bekannte Verbindungen (J. Amer. Chem. Soc. 76, 300, (1954)).

Die als Ausgangsverbindungen zur Durchführung des erfindungsgemäßen Verfahrens (b) erforderlichen Alkene der Formel (V) sind bekannt.

Die zur Durchführung des erfindungsgemäßen Verfahrens (c) als Ausgangsstoffe benötigten 1-Aryl-3-halogenalkyl-pyrazole sind bekannte Verbindungen der organischen Chemie (z.B. Chem. Lett. 1982, 543;).

Die zur Durchführung des erfindungsgemäßen Verfahrens (d) als Ausgangsstoffe benötigten N-Arylsydnone der Formel (VII) sind in Analogie zu bekannten Verfahren zugänglich (Or. Synth. 45, 96 (1965)).

Als Verdünnungsmittel zur Durchführung des erfindungsgemäßen Verfahrens (a) kommen organische Lösungsmittel infrage. Vorzugsweise werden polare organische Lösungsmittel verwendet, wie beispielsweise Eisessig, Ethanol, n-Butanol, Dimethylformamid oder Ethylenglykolmonomethylether. Zur Erleichterung des Ringschlusses zum Pyrazol kann man gegebenenfalls einen sauren Katalysator, wie beispielsweise Schwefelsäure, Salzsäure oder Trifluoressigsäure, vorzugsweise in geringen Mengen, zusetzen.

Die Reaktionstemperaturen können bei der Durchführung des erfindungsgemäßen Verfahrens (a) in einem größeren Bereich variiert werden. Im allgemeinen arbeitet man bei Temperaturen zwischen 0 °C und der Siedetemperatur des gegebenenfalls zugesetzten Verdünnungsmittels, vorzugsweise bei Temperaturen zwischen +20 °C und 120 °C.

Zur Durchführung des erfindungsgemäßen Verfahrens (a) setzt man pro Mol Acylvinylether der Formel (II) im allgemeinen 1,0 bis 2,0 Mol, vorzugsweise 1,0 bis 1,4 Mol, Arylhydrazin der Formel (III) ein. Die Reaktionsdurchführung und Reaktionskontrolle, die Aufarbeitung und Isolierung der Reaktionsprodukte erfolgt nach üblichen Methoden.

Eine besondere Variante des erfindungsgemäßen Verfahrens (a) besteht darin, daß man das Halogenierungsprodukt der Formel (X) in Gegenwart eines Arylhydrazins der Formel (III) dehydrohalogeniert.

Als Verdünnungsmittel zur Durchführung des erfindungsgemäßen Verfahrens (b) kommen inerte organische Lösungsmittel infrage.

Hierzu gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl oder -diethylether, Nitrile, wie Acetonitril oder Propionitril, Amide, wie Dimethylformamid, Dimethylacetamid, N-Methylformanilid, N-Methylpyrrolidon oder Hexamethylphosphorsäuretriamid oder Ester, wie Essigsäureethylester.

Das erfindungsgemäße Verfahren (b) wird vorzugsweise in Gegenwart eines geeigneten Säurebindemittels durchgeführt. Als solche kommen alle üblichen anorganischen oder organischen Basen infrage. Vorzugsweise verwendet man tertiäre Amine wie Triethylamin, N,N-Dimethylanilin, Pyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (b) in einem größeren Bereich variiert werden. Im allgemeinen arbeitet man bei Temperaturen zwischen -30 °C und +150 °C, vorzugsweise bei Temperaturen zwischen 0 °C und +100 °C.

Zur Durchführung des erfindungsgemäßen Herstellungsverfahrens (b) setzt man pro Mol N-Arylhydrazid-halogenid der Formel (IV) im allgemeinen 1,0 bis 20,0 Mol, vorzugsweise 1,0 bis 5,0 Mol, Alken der Formel (V) und gegebenenfalls 1,0 bis 20,0 Mol, vorzugsweise 1,0 bis 2,0 Mol, an Säurebindungsmittel ein. Die Reaktionsdurchführung und Reaktionskontrolle, die Aufarbeitung und Isolierung der Reaktionsprodukte der Formel (I) erfolgt nach allgemein bekannten Verfahren.

Als Verdünnungsmittel zur Durchführung des erfindungsgemäßen Verfahrens (c) kommen gegenüber den Halogenierungsmitteln inerte organische Lösungsmittel infrage. Beispielsweise eignen sich Tetrachlormethan, Fluorchlorkohlenwasserstoffe, wie z.B. Trichlorfluormethan 1,1,1-Trifluor-2,2,2-trichlorethan, 1,1,2-Trifluor-1,2,2,-trichlorethan, Eisessig, Ameisensäure, Trifluoressigsäure oder Perfluoralkane.

Die Reaktionstemperaturen können bei der Durchführung des erfindungsgemäßen Verfahrens (c) zwischen -78 °C und +100 °C variiert werden. Eine Fluorierung mit elementarem Fluor führt man vorzugsweise bei Temperaturen zwischen -78 °C und +20 °C durch, während eine Chlorierung bei höheren Temperaturen, z.B. bei der Temperatur des siedenden Tetrachlormethans, durchgeführt werden kann.

Die Aktivierung des Halogenierungsmittels kann durch Zugabe von Radikalinitiatoren, wie beispielsweise Azoisobutyronitril, oder durch UV-Licht vorgenommen werden.

Zur Durchführung des erfindungsgemäßen Verfahrens (c) setzt man pro Mol 1-Aryl-3-halogenalkylpyrazol der Formen (VI) 1,0 bis 3,0 Mol, vorzugsweise 1,0 bis 1,5 Mol, Halogenierungsmittel ein. Wiederum erfolgt die Kontrolle und Durchführung der Reaktion, die Aufarbeitung und Isolierung der Reaktionsprodukte nach bekannten Verfahren.

Als Verdünnungsmittel zur Durchführung des erfindungsgemäßen Verfahrens (d) eignen sich inerte organische Lösungsmittel, wie beispielsweise Toluol, Benzol, Xylol oder Mesitylen.

Die Reaktionstemperaturen bei der Durchführung dieses Verfahrens (d) hänge davon ab, ob das N-Arylsydnon der Formel (VII) thermisch oder photochemisch decarboxyliert wird. Im ersten Fall werden Temperaturen über 100 °C benötigt, die durch die Substituenten $R^2$ bis $R^6$ im N-Arylsydnon bestimmt werden. Für photochemisch induzierte Umsetzungen reichen Temperaturen unter 100 °C aus, vorzugsweise wird das Verfahren (d) in dem Temperturbereich zwischen -20 °C und 60 °C durchgeführt.

Zur Durchführung des erfindungsgemäßen Verfahrens (d) setzt man pro Mol N-Arylsydnon der Formel (VII) 1,0 bis 3,0 Mol Alkin der Formel (VIII), vorzugsweise 1,0 bis 2,0 Mol, der Verbindung (VIII) ein. Die Durchführung, Kontrolle und Aufarbeitung der Reaktion erfolgt nach an sich bekannten Verfahren.

Die Wirkstoffe der Formel (I) eignen sich zur Bekämpfung von tierischen Schädlingen, vorzugsweise Arthropoden und Nematoden, insbesondere Insekten und Spinnentieren, die in der Landwirtschaft, in Forsten, im Vorrats-und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscuc asellus, Armadillidium volgare, Porcellio scaber.

Aus der Ordnung der Diplopoda z.B. Blaniulus guttulatus.

Aus der Ordnung der Chiolopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp..

Aus der Ordnung der Anoplura z.B. Phylloxera vastatrix, Pemphigus spp., Pediculus humanus corporis, Haematopinus spp., Linoganthus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularuis, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumta, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis,

Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Acanthoscelides obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus holoeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp. Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stromoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..

Dabei lassen sich die erfindungsgemäßen Wirkstoffe mit besonders gutem Erfolg zur Bekämpfung von Blatt-und Bodeninsekten, wie beispielsweise gegen die Larven der Meerettichblattkäfer (Phaedon cochleariae) einsetzen.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Wirkstoff-imprägnierte Natur-und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt-und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte ali phatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder - schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo-und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsform kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

## Herstellungsbeispiele

### Beispiel 1

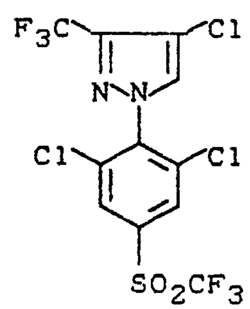

(Verfahren a)

10,1 g (0,05 Mol) 3-Chlor-4-ethoxy-1,1,1-trifluor-but-3-en-2-on und 15,5 g (0,05 Mol) 2,6-Dichlor-4-trifluormethylsulfonylphenylhydrazin werden in 50 ml Eisessig 6 Stunden auf 100 °C erwärmt. Nach Zugabe von Wasser extrahiert man mit Dichlormethan, wäscht die vereinigten Dichlormethanextrakte mit wäßriger Natriumbicarbonatlösung, trocknet über Natriumsulfat, filtriert und engt ein. Chromatographie an Kieselgel mit Petrolether (Siedebereich 60-80 °C) liefert 7,1 g (30 %) 4-Chlor-1-(2,6-dichlor-4-trifluormethylsulfonylphenyl)-3-trifluormethylpyrazol vom Schmelzpunkt 97-100 °C.

$^1$H-NMR (80 MHz, $CDCl_3$) : $\delta$ = 7,71 s (1H) und 8,13 ppm s (2H).

Beispiel 2

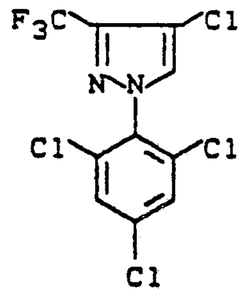

(Verfahren b)

Zu der Lösung von 11,1 g (0,03 Mol) 1-(2,4,6-Trichlorphenyl)-trifluoracethydrazidbromid und 5,82 g (0,06 Mol) 1,2-Dichlorethylen in 5 ml Toluol tropft man bei 80 °C 6,7 g (0,066 Mol) Triethylamin unter Rühren. Man rührt 5 Stunden bei 80 °C, kühlt ab und filtriert Triethylammoniumbromid ab. Einengen des Filtrats und Chromatographie des Rückstandes an Kieselgel mit Petrolether (Siedebereich 60-80 °C) liefert 1,44 g (14 %) 4-Chlor-1-(2,4,6-trichlorphenyl)-3-trifluormethylpyrazol vom Schmelzpunkt 79-83 °C.

$^1$H-NMR (80 MHz, $CDCl_3$) : $\delta$ = 7,50 s (2H) und 7,63 ppm s (1H).

Beispiel 3

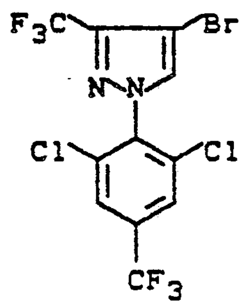

(Verfahren a)

8,5 g (0,05 Mol) 4-Ethoxy-1,1,1-trifluorbut-3-en-2-on in 50 ml n-Pentan werden bei -20 °C tropfenweise mit 8,0 g (0,05 Mol) Brom in 50 ml n-Pentan versetzt. Man rührt 1 Stunde bei -20 °C nach, destilliert n-Pentan ab und setzt 12,3 g (0,05 Mol) 2,6-Dichlor-4-trifluormethylphenylhydrazin in 100 ml Ethanol zu. Nach 2 Stunden Rückfluß engt man die Reaktionslösung im Wasserstrahlvakuum ein, nimmt den Rückstand in Dichlormethan auf, wäscht bis zur Neutralisation mit Wasser, trocknet über Natriumsulfat, filtriert und engt das Filtrat ein. Den Rückstand chromatographiert man an Kieselgel mit Toluol/Petrolether (Volumenverhältnis 1:1) und erhält 8,4 g (40 %) 4-Brom-1-(2,6-dichlor-4-trifluormethylphenyl)-3-trifluormethylpyrazol vom Schmelzpunkt 89-91 °C.

$^1$H-NMR (80 MHz, $CDCl_3$) : $\delta$ = 7,66 s (1H) und 7,74 ppm s (2H).

Beispiel 4

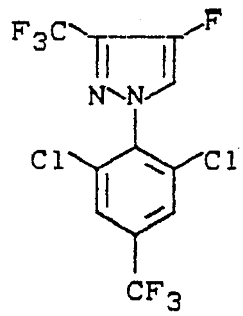

(Verfahren c)

In die Suspension von 3,0 g (0,8 mMol) 1-(2,6-Dichlor-4-trifluormethylphenyl)-3-trifluormethylpyrazol, 20 g Aluminiumoxid und 250 ml Fluortrichlormethan (Frigen® R11) leitet man bei -78 °C 2,8 g (10 mMol) Fluor als 5 % Lösung in Helium ein. Man rührt 30 Minuten bei -78 °C nach, läßt auf 0 °C erwärmen, trennt die Phasen und engt die organische Phase ein. Chromatographie an Kieselgel mit Petrolether (Siedebereich 60-80 °C) liefert 0,22 g 1-(2,6-Dichlor-4-trifluormethylphenyl)-4-fluor-3-trifluormethylpyrazol vom Siedepunkt 135 °C/0,075 Torr (Kugelrohr).

$^1$H-NMR (80 MHz, $CDCl_3$) : $\delta$ = 7,55 d (1H, J=4,1 Hz) und 7,75 ppm s (2H).

In entsprechender Weise und gemäß den allgemeinen Angaben zur Herstellung erhält man die in Tabelle 1 aufgeführten 1-Aryl-3-halogenalkyl-4-halogenpyrazole der allgemeinen Formel (I):

**Tabelle 1**

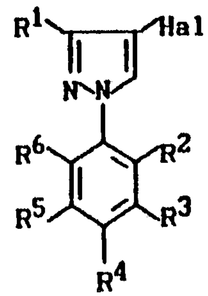

Allgemeine Formel (I)

| Bsp. Nr. | $R^1$ | Hal | $R^2$ | $R^3$ | $R^4$ | $R^5$ | $R^6$ | Ausbeute (%) | Schmp. (°C) | NMR (80 MHz, $CDCl_3$, δ-Werte) |
|---|---|---|---|---|---|---|---|---|---|---|
| 5 | $CF_3$ | Cl | Cl | Cl | $CF_3$ | H | Cl | 29 | 99-101 | 7,65 s (1H), 7,86 s (1H) |
| 6 | $CF_3$ | Cl | Cl | H | $CF_3$ | H | Cl | 23 | 85-86 | 7,65 s (1H), 7,75 s (1H) |
| 7 | $CF_3$ | Br | Cl | H | Cl | H | Cl | 31 | 93-94 | 7,49 s (2H), 7,65 s (1H) |
| 8 | $CF_3$ | F | Cl | H | $SO_2CF_3$ | H | Cl | 51 | 103-106 | 8,08 s (2H), 7,55 s (1H, J=4,1 Hz) |

Herstellung der Ausgangsverbindungen

Beispiel 9

Zu der Lösung von 25,5 g (0,15 Mol) 4-Ethoxy-1,1,1-trifluorbut-3-en-2-on in 150 ml Dichlormethan werden bei 0°C 21,0 g (0,165 Mol) Sulfurylchlorid in 90 ml Dichlormethan unter Rühren innerhalb von 30 Min. getropft. Man rührt 3 Stunden bei 0°C nach, destilliert das Lösungsmittel und nichtumgesetztes Sulfurylchlorid im Wasserstrahlvakuum weitgehend ab und erhält 38,3 g hellgelbes Öl. Die Konstitution folgt aus dem $^1$H-NMR-Spektrum ($CDCl_3$), das im Einklang mit der Struktur eines aus etwa gleichen Teilen bestehenden Gemisches der diastereomeren 3,4-Dichlor-4-ethoxy-1,1,1-trifluorbutan-2-one Signale zwischen 1,1-1,5, 3,5-4,2, 4,75-5,0 und 5,75-5,9 ppm im Verhältnis 3:2:1:1 zeigt. Der Anteil an 3-Chlor-4-ethoxy-1,1,1-trifluorbut-3-en-2-on im Gemisch beträgt weniger als 5 %. Durch Destillieren bei 1 Torr läßt sich das Diastereomerengemisch unzersetzt von Lösungsmittelresten befreien.

Beispiel 10

Das nach Beispiel 9 erhaltene 3,4-Dichlor-4-ethoxy1,1,1-trifluorbutan-2-on (38,0 g) wird langsam im Wasserstrahlvakuum destilliert und liefert 23,9 g schwach gelb gefärbtes 3-Chlor-4-ethoxy-1,1,1-trifluor-but-3-en-2-on vom Siedepunkt 84-87°C/10 Torr, $n_D^{20}$ = 1,4380.

$^1$H-NMR ($CDCl_3$) : $\delta$ = 1,55 t (3H, J=8 Hz) 4,40 q (2H) und 8,00 ppm s (1H)

IR ($CCl_4$): $\nu_{CO}$ = 1710 $cm^{-1}$.

Beispiel 11

Zu 8,35 g (0,05 Mol) 4-Ethoxy-1,1,1-trifluor-but-3-en-2-on in 50 ml Pentan werden bei -20°C unter Rühren 8,0 g (0,05 Mol) Brom in 50 ml Pentan getropft. Man rührt 1 Stunde bei -20°C nach und tropft anschließend die Lösung von 6,0 g (0,06 Mol) Triethylamin in 150 ml Pentan zu. Nach Rühren über Nacht bei 20°C wird der Reaktionsansatz auf Eis gegeben. Man säuert mit 1n Salzsäure an (pH-Wert 4-5) und extrahiert mit Petrolether. Trocknen über wasserfreiem Natriumsulfat und Eindampfen der Lösung liefert 12,1 g gelbliche Flüssigkeit, die fraktionierend unter vermindertem Druck destilliert wird. 10,4 g 3-Brom-4-ethoxy-1,1,1-trifluorobut-3-en-2-on vom Siedepunkt 100-120°C/16 Torr, $n_D^{20}$ = 1,4680 werden erhalten, die bei -30°C kristallisieren.

$^1$H-NMR ($CDCl_3$) : $\delta$ = 1,51 t (3H, J=8 Hz) 4,45 q (2H) und 8,06 ppm s (1H)

IR ($CCl_4$): $\nu_{CO}$ = 1710 $cm^{-1}$.

Beispiel 12

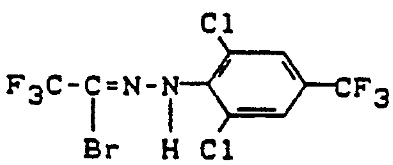

Zu einer Lösung von 8,12 g (0,025 Mol) Trifluoracetaldehyd N-(2,6-Dichlor-4-trifluormethylphenyl)-hydrazon in 7,5 ml Dimethylformamid fügt man bei Raumtemperatur portionsweise 4,45 g (0,025 Mol) N-Bromsuccinimid, wobei eine exotherme Reaktion auftritt. Man rührt 3 Stunden bei Raumtemperatur, rotiert das Dimethylformamid ab und versetzt den Rückstand mit 20 ml Petrolether. Nach Absaugen des ausgefallenen Succinimids wird das Filtrat eingeengt und einer Kugelrohrdestillation unterworfen. Man erhält

9,26 (91,7 %) N-(2,6-Dichlor-4-trifluormethylphenyl)-trifluoracethydrazid-bromid. Siedepunkt 110 °C (0,05 Torr, $n_D^{20}$ 1,510.

$^1$H-NMR (80 MHz, $CDCl_3$, δ-Werte): 8,56 (bs,1H); 7,74 (s, 2H).

Beispiel 13

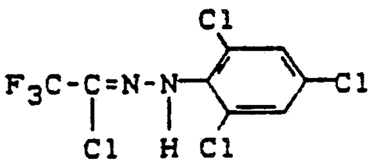

3,2 g (0,011 Mol) Trifluoracetaldehyd N-(2,4,6-Trichlorphenyl)-hydrazon und 1,5 g (0,011 Mol) N-Chlorsuccinimid in 3,3 ml Dimethylformamid werden 3 Stunden bei Raumtemperatur gerührt. Nach Einengen versetzt man mit Petrolether, filtriert und engt wieder ein. Kugelrohrdestillation des Rückstandes bei 150 °C (0,35 Torr) liefert 3,28 g (89,6 %) N-(2,4,6-Trichlorphenyl)-trifluoracethydrazid-chlorid, $_D^{20}$ 1,549.

$^1$H-NMR (80 MHz, $CDCl_3$, δ-Werte): 8,12 (bs,1H); 7,48 (s, 2H).

In entsprechender Weise und gemäß den allgemeinen Herstellungsangaben erhält man die in der folgenden Tabelle 2 aufgeführten N-Aryl-halogenalkyl-hydrazid-halogenide der allgemeinen Formel (IV):

Tabelle 2

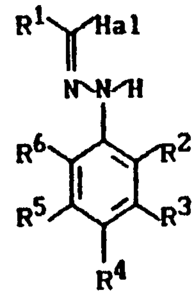

Allgemeine Formel (IV)

| Bsp. Nr. | Hal | $R^1$ | $R^3$ | $R^4$ | $R^5$ | $R^6$ | $R^7$ | Ausbeute (%) | Schmp. (°C) Sdp. (°C/Torr) | $^1$H-NMR (80 MHz, $CDCl_3$, δ-Werte) |
|---|---|---|---|---|---|---|---|---|---|---|
| 14 | Br | $F_3C$ | Cl | H | Cl | H | Cl | 94 | 125 (0,06) | 8,20 (bs,1H), 7,47 (s,2H) |
| 15 | Cl | $F_3C$ | Cl | H | $F_3C$ | H | Cl | 87 | 130 (0,4) | 8,47 (bs,1H); 7,72 (s,2H) |
| 16 | Br | $F_3C$ | Cl | H | $F_3C$ | H | Cl | 91 | 62-63 | 8,48 (bs,1H); 7,71 (s,1H) |
| 17 | Br | $F_3C$ | Cl | H | $F_3C$ | H | H | 95 | 95 (0,04) | 8,68 (bs,1H); 7,65-7,45 (m,3H) |
| 18 | Cl | $F_3C$ | Cl | H | $F_3C$ | H | H | 84 | 90 (0,1) | 8,62 (bs,1H); 7,65-7,48 (m,3H) |
| 19 | Br | $F_3C$ | Cl | H | $F_3C-SO_2$ | H | Cl | 83 | 48-50 | 8,82 (bs,1H); 7,99 (s,2H) |

Beispiel 20

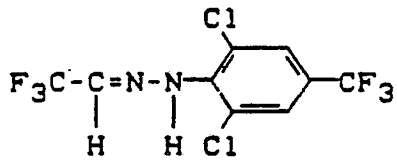

Ein Gemisch von 100 g (0,69 Mol) Trifluoracetaldehydeethylsemiacetal und 169,1 g (0,69 mol) 2,6-Dichlor-4-trifluormethyl-phenylhydrazin wird 6 Stunden bei 100 °C erhitzt. Nach Entfernung der flüchtigen Komponenten wird der Rückstand aus Petrolether umkristallisiert. Man isoliert 200 g (89 %) Trifluoracetaldehyd N-(2,6-Dichlor-4-trifluormethylphenyl)-hydrazon vom Schmelzpunkt 45 bis 46 °C.

$^1$H-NMR (80 MHz, $CDCl_3$, δ-Werte): 8,15 (bs,1H); 7,72 (s, 1H); 7,12 (q,1H, J = 3,8 Hz).

In entsprechender Weise und gemäß den allgemeinen Angaben zur Herstellung erhält man die in der folgenden Tabelle 3 aufgeführten Halogenalkylaldehyd-N-arylhydrazone der allgemeinen Formel (XIII):

Tabelle 3

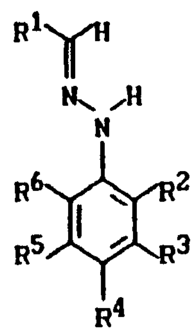

Allgemeine Formel (XIII)

| Bsp. | $R^1$ | $R^3$ | $R^4$ | $R^5$ | $R^6$ | $R^7$ | Ausb. (%) | Schmp. (°C) | $^1$H-NMR (80 MHz, $CDCl_3$, δ-Werte) |
|---|---|---|---|---|---|---|---|---|---|
| 21 | $F_3C$ | Cl | H | Cl | H | Cl | 92 | 43-45 | 7,85 (bs,1H); 7,56 (s,1H); 6,88 (q,1H, J=3,9 Hz) |
| 22 | $F_3C$ | Cl | Cl | $F_3C$ | H | Cl | 90 | 50-52 | 8,13 (bs,1H); 7,69 (s,1H); 7,08 (q,1H), J=4 Hz) |
| 23 | $F_3C$ | Cl | H | $F_3C$ | H | H | 89 | 63-65 | 8,48 (bs,1H; 7,61 (d,1H); 7,58 (s,1H); 7,49 (d,1H); 7,17 (q,1H; J=4 Hz) |
| 24 | $F_3C$ | Cl | H | $F_3C-SO_2$ | H | Cl | 80 | 124-126 | 8,54 (bs,1H); 7,95 (s,1H); 7,25 (q,1H, J=4 Hz) |

Anwendungsbeispiele

In den folgenden Anwendungsbeispielen wurde die nachstehend aufgeführte Verbindung als Vergleichsverbindung eingesetzt:

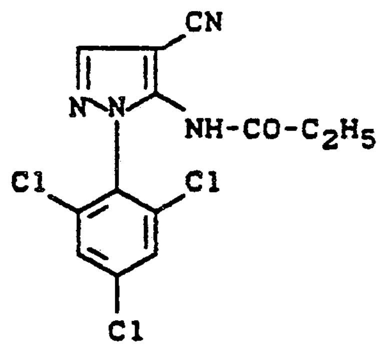

(A)

4-Cyano-5-propionylamino-1-(2,4,6-trichlorphenyl)-pyrazol (bekannt aus DE-OS 3 226 513).

Beispiel A

$LD_{100}$-Test

Testtiere: Sitophilus granarius
Lösungsmittel: Aceton

2 Gewichtsteile Wirkstoff werden in 1.000 Volumenteilen Lösungsmittel aufgenommen. Die so erhaltene Lösung wird mit weiteren Lösungsmitteln auf die gewünschte Konzentration verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro $m^2$ Filterpapier verschieden hoch. Anschließend gibt man etwa 25 Testtiere in die Petrischale und bedeckt sie mit einem Glasdeckel.

Der Zustand der Testtiere wird 3 Tage nach Ansetzen der Versuche kontrolliert. Bestimmt wird die Abtötung in %.

Bei diesem Test zeigen z. B. die folgenden Verbindungen der Herstellungsbeispiele überlegene Wirkung gegenüber dem Stand der Technik: (1), (2), (3), (4), (6), (7).

Beispiel B

$LD_{100}$-Test

Testtiere: Blattella germanica
Lösungsmittel: Aceton

2 Gewichtsteile Wirkstoff werden in 1.000 Volumenteilen Lösungsmittel aufgenommen. Die so erhaltene Lösung wird mit weiteren Lösungsmitteln auf die gewünschten Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro $m^2$ Filterpapier verschieden hoch. Anschließend gibt man 10 Testtiere in die Petrischale und bedeckt sie mit einem Glasdeckel.

Der Zustand der Testtiere wird 3 Tage nach Ansetzen der Versuch kontrolliert. Bestimmt wird die Abtötung in %.

Bei diesem Test zeigen z. B. die folgenden Verbindungen der Herstellungsbeispiele überlegene Wirkung gegenüber dem Stand der Technik: (1), (2), (3), (4), (6).

Beispiel C

$LT_{100}$-Test für Dipteren

Testtiere: Musca domestica
Zahl der Testtiere: 25
Lösungsmittel: Aceton

2 Gewichtsteile Wirkstoff werden in 1.000 Volumenteilen Lösungsmittel aufgenommen. Die so erhaltene Lösung wird mit weiterem Lösungsmittel auf die gewünschten geringeren Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro $m^2$ Filterpapier verschieden hoch. Anschließend gibt man die angegebene Anzahl der Testtiere in die Petrischale und bedeckt sie mit einem Glasdeckel.

Der Zustand der Testtiere wird laufend kontrolliert. Es word diejenige Zeit ermittelt, welche für einen 100 % igen knock down-Effekt notwendig ist.

Bei diesem Test zeigen z. B. die folgenden Verbindungen der Herstellungsbeispiele überlegene Wirkung gegenüber dem Stand der Technik: (1), (2), (3), (4), (6).

Beispiel D

Phaedon-Larven-Test

Lösungsmittel: 7 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100 %, daß alle Käfer-Larven abgetötet wurden; 0 % bedeutet, daß keine Käfer-Larven abgetötet wurden.

Bei diesem Test zeigen z. B. die folgenden Verbindungen der Herstellungsbeispiele überlegene Wirksamkeit gegenüber dem Stand der Technik: (1), (2), (3), (5), (6), (7).

## Ansprüche

1. 1-Aryl-3-halogenalkyl-4-halogen-pyrazole der Formel (I)

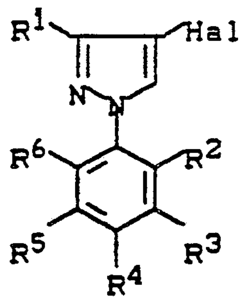

(I)

in welcher
$R^1$ für Halogenalkyl steht,
Hal für Halogen steht und
$R^2$, $R^3$, $R^4$, $R^5$ und $R^6$ jeweils unabhängig voneinander für Wasserstoff, Halogen, Cyano, Nitro, Alkyl, Alkylthio, Alkylsulfinyl, Alkylsulfonyl, Alkoxycarbonyl oder für den Rest $-(X)_n-R^7$ stehen,
wobei
X für Sauerstoff, Schwefel, Sulfinyl oder Sulfonyl steht,
n für die Zahl 0 oder 1 steht und
$R^7$ für Halogenalkyl steht,
wobei, falls $R^2$ bis $R^6$ gleichzeitig für Wasserstoff stehen, $R^1$ nicht für Trifluormethyl und Hal nicht für Fluor stehen darf.

2. 1-Aryl-3-halogenalkyl-4-halogen-pyrazole der Formel (I) gemäß Anspruch 1, dadurch gekennzeichnet, daß
$R^1$ für geradkettiges oder verzweigtes Halogenalkyl mit 1 bis 4 Kohlenstoffatomen und 1 bis 9 gleichen oder verschiedenen Halogenatomen steht,
Hal für Fluor, Chlor, Brom oder Iod steht und
$R^2$, $R^3$, $R^4$, $R^5$ und $R^6$ jeweils unabhängig voneinander für Wasserstoff, Halogen, Cyan, Nitro, Alkyl, Alkylthio, Halogenalkyl, Halogenalkoxy, Halogenalkylthio, Alkylsulfinyl, Alkylsulfonyl, Halogenalkylsulfinyl, Halogenalkylsulfonyl oder Alkoxycarbonyl mit jeweils 1 bis 4 Kohlenstoffatomen in den einzelnen Alkylteilen und gegebenfalls 1 bis 9 gleichen oder verschiedenen Halogenatomen stehen, wobei, falls $R^2$ bis $R^6$ gleichzeitig für Wasserstoff stehen $R^1$ nicht für Trifluormethyl und Hal nicht für Fluor stehen darf.

3. 1-Aryl-3-halogenalkyl-4-halogen-pyrazole der Formel (I) gemäß Anspruch 1 und 2, dadurch gekennzeichnet, daß
$R^1$ für Trifluormethyl, Dichlorfluormethyl, Difluorchlormethyl, Difluormethyl, Trifluorethyl, Trifluorchlorethyl, Tetrafluorethyl, Pentafluorethyl, Heptafluorpropyl oder Nonafluorbutyl steht,
Hal für Fluor, Chlor oder Brom steht und
$R^2$, $R^3$, $R^4$, $R^5$ und $R^6$ jeweils unabhängig voneinander für Wasserstoff, Fluor, Chlor, Brom, Cyan, Nitro, Methyl, Ethyl, n-oder i-Propyl, n-, i-, s-oder tert.-Butyl, Methylthio, Methylsulfinyl, Methylsulfonyl, Methoxycarbonyl, Ethoxycarbonyl, Trifluormethyl, Dichlorfluormethyl, Difluorchlormethyl, Difluormethyl, Pentafluorethyl, Trifluorchlorethyl, Trifluorethyl, Trifluordichlorethyl, Difluorchlorethyl oder für den Rest $-X-R^7$ steht,
wobei
X für Sauerstoff, Schwefel, Sulfinyl oder Sulfonyl steht und
$R^7$ für Trifluormethyl, Dichlorfluromethyl, Difluorchlormethyl, Difluormethyl, Pentafluorethyl, Tetrafluorethyl, Trifluorchlorethyl, Trifluorethyl, Difluorchlorethyl, Trichlormethyl oder Trifluordichlorethyl steht,
wobei, falls $R^2$ bis $R^6$ gleichzeitig für Wasserstoff stehen, $R^1$ nicht für Trifluormethyl und Hal nicht für Fluor stehen darf.

4. Verfahren zur Herstellung von 1-Aryl-3-halogenalkyl-4-halogen-pyrazolen der Formel (I)

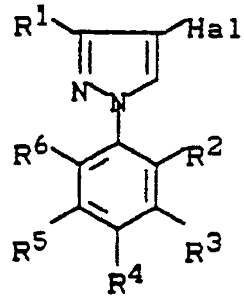

(I)

in welcher
$R^1$ für Halogenalkyl steht,
Hal für Halogen steht und
$R^2$, $R^3$, $R^4$, $R^5$ und $R^6$ jeweils unabhängig voneinander für Wasserstoff, Halogen, Cyano, Nitro, Alkyl, Alkylthio, Alkylsulfinyl, Alkylsulfonyl, Alkoxycarbonyl oder für den Rest $-(X)_n-R^7$ stehen,
wobei
X für Sauerstoff, Schwefel, Sulfinyl oder Sulfonyl steht,
n für die Zahl 0 oder 1 steht und
$R^7$ für Halogenalkyl steht,
wobei, falls $R^2$ bis $R^6$ gleichzeitig für Wasserstoff stehen, $R^1$ nicht für Trifluormethyl und Hal nicht für Fluor stehen darf,
dadurch gekennzeichnet, daß man

a) halogensubstituierte Acylenolether der Formel (II)

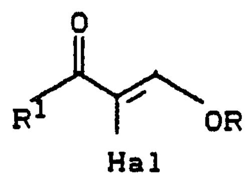

(II)

in welcher
$R^1$ für Halogenalkyl steht,
Hal für Halogen steht und
R für Alkyl steht,
mit Arylhydrazinen der Formel (III)

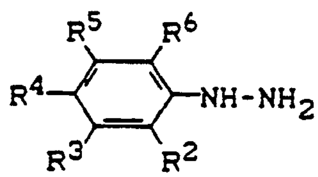

(III)

in welcher
$R^2$ bis $R^6$ die obengenannte Bedeutung haben, gegebenenfalls in einem Verdünnungsmittel umsetzt, oder

b) N-Arylhydrazid-halogenide der Formel (IV)

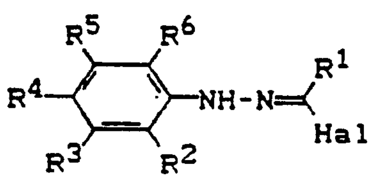

(IV)

in welcher
$R^1$ bis $R^6$ und Hal die obengenannte Bedeutung haben, mit Alkenen der Formel (V)

Hal-CH = CH-Hal (V)

in welcher
Hal für Halogen steht, gegebenenfalls in einem Verdünnungsmittel und gegebenenfalls in Gegenwart eines säurebindenden Mittels umsetzt, oder

c) 1-Aryl-3-halogenalkyl-pyrazole der Formel (VI)

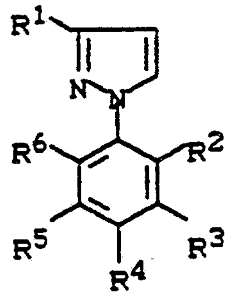

(VI)

in welcher
$R^1$ bis $R^6$ die obengenannte Bedeutung haben,
mit einem Halogenierungsmittel, gegebenenfalls in einem Verdünungsmittel umsetzt, oder

d) N-Arylsydnone der Formel (VII)

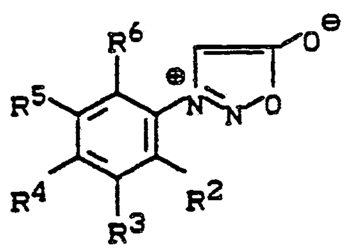

(VII)

in welcher
$R^2$ bis $R^6$ die obengenannte Bedeutung haben, mit Alkinen der Formel (VIII)

$R^1$-C≡C-Hal (VIII)

in welcher
$R^1$ und Hal die genannte Bedeutung haben,
gegebenenfalls in einem Verdünnungsmittel umsetzt.

5. Insektizide Mittel, gekennzeichnet, durch einen Gehalt an mindestens einem 1-Aryl-3-halogenalkyl-4-halogen-pyrazol der Formel (I).

6. Verfahren zur Bekämpfung von Insekten, dadurch gekennzeichnet, daß man 1-Aryl-3-halogenalkyl-4-halogen-pyrazole der Formel (I) auf Insekten und/oder deren Lebensraum einwirken läßt.

7. Verwendung von 1-Aryl-3-halogenalkyl-4-halogenpyrazolen der Formel (I) zur Bekämpfung von Insekten.

8. Verfahren zur Herstellung von insektiziden Mitteln, dadurch gekennzeichnet, daß man 1-Aryl-3-halogenalkyl-4-halogen-pyrazole der Formel (I) mit Streckmitteln und/oder oberflächenaktiven Mitteln vermischt.

Europäisches Patentamt

# EUROPÄISCHER RECHERCHENBERICHT

Nummer der Anmeldung

EP 88 10 7011

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,X | JOURNAL OF THE CHEMICAL SOCIETY PERKIN TRANSACTIONS I, Nr. 9, 22. Januar 1982, Seiten 2207-2210, London, GB; G.B. BLACKWELL et al.: "Polyhalogeno-allenes and -acetylenes. Part 15. Dipolar cycloadditions of N-phenylsydnone and aryl azides to perfluoropropadiene and perfluoropropyne" * Das ganze Dokument * --- | 1-4 | C 07 D 231/16<br>A 01 N 43/56 |
| A | EP-A-0 202 169 (HOKKO CHEMICAL INDUSTRY) * Das ganze Dokument * --- | | |
| A | EP-A-0 138 527 (NIPPON K.K.) * Das ganze Dokument * --- | | |
| P,A | EP-A-0 242 322 (SANDOZ) * Das ganze Dokument * ----- | | |
| | | | RECHERCHIERTE SACHGEBIETE (Int. Cl.4) |
| | | | C 07 B 231/00<br>A 01 N 43/00 |

Der vorliegende Recherchenbericht wurde für alle Patentansprüche erstellt

| Recherchenort | Abschlußdatum der Recherche | Prüfer |
|---|---|---|
| DEN HAAG | 02-08-1988 | DE BUYSER I.A.F. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)